# EUROPEAN PATENT SPECIFICATION

(11) **EP 1 501 480 B1**
(45) Date of publication and mention of the grant of the patent: **26.05.2010**
(21) Application number: 03716960.4
(22) Date of filing: 31.03.2003
(51) Int. Cl.: A61K 9/127

(54) **METHODS AND FORMULATIONS FOR ENHANCING THE ABSORPTION AND GASTRO-INTESTINAL BIOAVAILABILITY OF HYDROPHOBIC DRUGS**
VERFAHREN UND ZUSAMMENSETZUNG ZUR ERHÖHUNG DER ABSORPTION UND DER BIOVERFÜGBARKEIT VON HYDROPHOBEN WIRKSTOFFEN IM MAGEN-DARMTRAKT
METHODES ET FORMULATIONS DESTINEES A FAVORISER L'ABSORPTION ET LA BIODISPONIBILITE GASTRO-INTESTINALE DE MEDICAMENTS HYDROPHOBES

(30) Priority: 07.05.2002 US 140620
(43) Date of publication of application: 02.02.2005
(73) Proprietor: Zomanex, LLC, Chesterfield, MO 63017 (US)
(72) Inventor: SPILBURG, Curtis A., Kapac, LLC, Chesterfield, MO 63017 (US)
(74) Representative: Tomkinson, Alexandra
(86) International application number: PCT/US2003/010146
(87) International publication number: WO 2003/094891

(56) References cited:
- EP-A- 0 736 299
- WO-A1-94/28876
- US-A- 4 311 712
- US-A- 4 508 703
- US-A- 5 202 126
- US-A- 5 290 562

## Description

### FIELD OF THE INVENTION

This invention relates to a drug-delivery system for enhancing the bioavailability of hydrophobic drug active compounds, using naturally-occurring formulation ingredients that are present in the diet. Specifically, this invention is especially useful as a general formulation for the delivery of drugs in dry form that heretofore have produced variable pharmacological responses, which are indicative of poor bioavilability.

### BACKGROUND OF THE INVENTION

Many drugs are absorbed by passive diffusion through a hydrophobic cellular membrane, which does not participate in the absorption process. The amount of absorbed drug is controlled by two processes. First, a high concentration of the active substance at the membrane surface will enhance cellular absorption (Fick's Law). Since cells function in an aqueous environment, enhancing the water solubility of a drug increases its concentration at the locus of absorption. However, while greater water solubility may be expected to enhance the bioavailability of drugs, this is frequently not the case due to a second, competing process that affects the overall absorption process. The absorptive cell membrane is composed mainly of lipids that prevent the passage of hydrophilic compounds, but which are highly permeable to lipid soluble substances. Therefore, the design of bioavailable drugs must balance two opposing forces. On the one hand, a drug that is very hydrophilic may have a high concentration at the cell surface but be impermeable to the lipid membrane. On the other hand, a hydrophobic drug that may easily "dissolve" in the membrane lipids may be virtually insoluble in water producing a very low concentration of the active substance at the cell surface.

To circumvent these problems, a number of strategies have been used to maintain the hydrophobicity of the drug and at the same time to provide a "packaging" matrix that increases its aqueous concentration. For example, emulsions can be prepared for the parenteral delivery of drugs dissolved in vegetable oil [Collins-Gold, L., Feichtinger, N. & Warnheim, T. (2000) "Are lipid emulsions the drug delivery solution?" Modern Drug Discovery, 3, 44-46.] Alternatively, artificial membranes or liposomes have been used to encapsulate a variety of drugs for different delivery routes, including oral, parenteral and transdermal [Cevc, G. and Paltauf, F., eds., "Phospholipids: Characterization, Metabolism, and Novel Biological Applications", pp. 67-79, 126-133, AOCS Press, Champaign, IL, 1995]. All these methods require amphiphiles, compounds that have a hydrophilic or polar end and a hydrophobic or nonpolar end, such as phospholipid, cholesterol or glycolipid.

When amphiphiles are added to water, they form lipid bilayer structures (liposomes) that contain an aqueous core surrounded by a hydrophobic membrane. This novel structure can deliver water insoluble drugs that are "dissolved" in its hydrophobic membrane or, alternatively, water soluble drugs can be encapsulated within its aqueous core. This strategy has been employed in a number of fields. For example, liposomes have been used as drug carriers since they are rapidly taken up by the cells and, moreover, by the addition of specific molecules to the liposomal surface they can be targeted to certain cell types or organs, an approach that is typically used for drugs that are encapsulated in the aqueous core. For cosmetic applications, phospholipid and lipid substances are dissolved in organic solvent and, with solvent removal, the resulting solid may be partially hydrated with water and oil to form a cosmetic cream or drug-containing ointment. Finally, liposomes have been found to stabilize certain food ingredients, such as omega-3 fatty acid-containing fish oils to reduce oxidation and rancidity (Haynes et al, U.S. Patent 5,139,803).

In an early description of liposome formulation (Bangham et al., 1965 J. Mol. Biol. 13, 238-252), multilammelar vesicles were prepared by the addition of water and mechanical energy to the waxy film that was formed by removing the organic solvent that was used to dissolve the phospholipid. In later work, it was found that the combination of sterols (cholesterol, phytosterols) and phospholipid allowed the formulation of liposomes with more desirable properties, such as enhanced stabilization and encapsulation efficiency. The patent and scientific literature describe many methodological improvements to this general strategy. However, none presently known achieves the efficient delivery rates of the present invention.

Even though liposomes provide an elegant method for drug delivery, their use has been limited by cumbersome preparation methods and the inherent instability of aqueous preparations. A number of patents describe the large scale preparation of pre-liposomal components that can be hydrated later to form the desired aqueous-based delivery vehicle. Evans 35 al. (U.S. Patent 4,311,712) teach that all the components (phospholipid, cholesterol and biological agent) can be mixed in an organic solvent with a melting point near that of room temperature. After solvent removal by lyophilization, addition of water produced liposomes with the biologically active material "dissolved" in the membrane. Similarly, U.S. Patent 5,202,126 (Perrier et al.) teaches the addition of all the components in the organic phase, but with solvent removal accomplished by atomization following the method described by Redziniak et al. (U.S. Patents 4,508,703 and 4,621,023). The pulverulent solid so produced can then be hydrated, homogenized and converted into a cream for the topical delivery of the biologically active material, in this case pregnenolone or pregnenolone ester. Orthoefer describes the preparation of liquid crystal phospholipid (U.S. Patent 6,312,703) as a novel carrier for biologically active compounds. In this method, the various solid components are pre-mixed and then subjected to high pressure to form a lecithin bar that can be used in cosmetic applications as soap or the pressurized components can be extruded as a rope and cut into pharmaceutical-containing tablets. Unlike previous work, this method does not teach premixing in organic solvent or homogenization in water.

The utility of a dried preparation to enhance the stability and shelf life of the liposome components has long been recognized, and numerous methods have been devised to maintain the stability of liposomal preparations under drying conditions. Schneider (U.S. Patent 4,229,360) describes the preparation of encapsulated insulin in liposomes by adding the aqueous peptide solution to a film of phospholipid. Lyophilization of this liposomal mixture in the presence of gum arabic or dextran produced a solid that could be reconstituted with water to form liposomes. However, following a similar procedure to encapsulate cyclosporin, Rahman et al. (4,963,362) teach that the lyophilization step can be performed without the addition of other additives, such that the re-hydrated liposomes maintain their ability to encapsulate the bioactive substance. Vanlerberghe et al. (U.S. Patent 4,247,411) teach a similar process, but include sterols with the phospholipid to provide a more stable liposome. In an effort to enhance the stability and dispersibility of liposomes in a solid matrix, Payne et al. (U.S. Patents 4,744,989 and 4,830,858) describe methods for coating a water soluble carrier, such as dextrose, with a thin film of liposome components. When added to water, the carrier dissolves and the liposome components hydrate to form liposomes.

The goal of all these methods is to produce a solid that can be re-hydrated at a later time to form liposomes that can deliver a biologically active substance to a target tissue or organ. Surprisingly, there have been only two reports that use the dried liposome preparations themselves, with no intermediate hydration, as the delivery system. Ostlund, U.S. Patent 5,932,562 teaches the preparation of solid mixes of plant sterols for the reduction of cholesterol absorption. Plant sterols or plant stenols are premised with lecithin or other amphiphiles in organic solvent, the solvent removed and the solid added hack to water and homogenized. The emulsified solution is dried and dispersed in foods or compressed into tablets or capsules. In this case, the active substance is one of the structural components of the liposome itself (plant sterol) and no additional biologically active substance was added. Manzo et al. (US Patent 6,083,529) teach the preparation of a stable dry powder by spray drying an emulsified mixture of lecithin, starch and an anti-inflammatory agent. When applied to the skin, the biologically active moiety is released from the powder only in the presence of moisture. Neither Ostlund nor Manzo suggest or teach the use of sterol, and lecithin and a drug active, all combined with a non-polar solvent and then processed to provide a dried drug carrying liposome of enhanced delivery rates.

Mazzei et al (EP0736299B1) discloses a composition that contains a liposomal water dispersible orally administered drug blend containing a lipid of natural origin, cyclosporine and a sterol.

Substances other than lecithin have been used as dispersing agents. Following the same steps (dissolution in organic solvent, solvent removal, homogenization in water and spray drying) as those described in U.S Patent 5,932,562, Ostlund teaches that the surfactant sodium steroyl lactylate can be used in place of lecithin (U.S Patent 6,063,776). Burruano et al. (U.S. Patents 6,054,144 and 6,110,502) describe a method of dispersing soy sterols and stanols or their organic esters in the presence of a mono-functional surfactant and a poly-functional surfactant without homogenization. The particle size of the solid plant-derived compounds is first reduced by milling and then mixed with the surfactants in water. This mixture is then spray dried to produce a solid that can be readily dispersed in water.
Similarly, Bruce et al. (U.S. Patent 6,242,001) describe the preparation of melts that contain plant sterols/stanols and a suitable hydrocarbon.

On cooling these solids can be milled and added to water to produce dispersible sterols. Importantly, none of these methods anticipate the type of delivery method described here as a means to deliver hydrophobic, biologically active compounds.

All the above described art, either deals with lowering of cholesterol of with a variety of techniques used in an attempt to solubilise some hydrophobic drugs using specific lipids. None teach or suggest a generalised approach to both enhance solubilisation in a water environment and enhance the rate of diffusion of hydrophobic drugs through lipid membranes of cell walls so that the drug has an increased bio availability at any given dose.

An object of the invention is to enhance the biological activity of a hydrophobic drug substance by its "dispersibility" through the use of a combination of amphiphiles.

### Summary of the Invention

According to the present invention there is provided a solid drug delivery composition for normally difficulty soluble hydrophobic drug actives, comprising: lecithin or an active derivative thereof;
a plant derived sterol (stanol) or ester derived from the sterol (stanol); and
an effective amount of a hydrophobic drug active,
wherein the weight ratio of lecithin or active derivative of lecithin to the combination of the sterol (stanol) or ester derived from the sterol (stanol) and drug active is from 0.20:1.0 to 1:1,
said composition being solid.

A general method and delivery composition is provided for enhancing the bioavailability of hydrophobic, poorly water soluble compounds and drugs, using the following steps and materials:
a) Phospholipid, such as lecithin or one of its derivatives, a sterol (preferably a plant-derived sterol and most preferably a reduced plant-derived sterol) and a selected drug are mixed in a non-polar solvent (preferably ethyl acetate or heptane) at its boiling point;
b) a solid residue is collected after the solvent is driven off at elevated temperature to maintain the solubility of all the components;
c) the solid residue is broken into small pieces and dispersed with vigorous stirring in water to form a milky solution at a temperature that is less than the decomposition temperature of any one of the components or the boiling point of water, whichever is lower;
d) rhe milky solution is passed through a homogeniser, such as a Gaulin Dairy Homogeniser (or suitable equivalent) operating at a maximum pressure; and thereafter
e) a suitable drying aid is added (e.g. Maltrin, Capsule M or suitable equivalent and then the milky solution is spray dried or lyophilized to produce a solid that can be incorporated into tablets or capsules, providing the appropriate excipients are added.

In another alternative method, lecithin, plant sterols and active drug are mixed in the presence of an organic solvent such as hexane or ethyl acetate, the solvent removed and the solid compressed and extruded for the formulation of tablets and capsules.

The formulation method described contains a minimum of three components, a lecithin emulsifier, a sterol and a hydrophobic active or drug compound, all of which must be soluble in an organic solvent.

### DETAILED DESCRIPTION OF A PREFERRED EMBODIMENT

Numerous amphiphilic emulsifiers have been described, but since this invention contemplates pharmaceutical application only those compounds that have been approved for human use are acceptable. The emulsifier used in the present invention is lecithin derived from egg yolk, soy beans or any of its chemically modified derivatives, such as lysolecithin. Lecithin is not only an excellent emulsifier and surfactant, it also has many health benefits that are beneficial when used as the contemplated pharmaceutical formulation agent described here [Cevc, G. and Paltauf, F., eds., "Phospholipids: Characterization, Metabolism, and Novel Biological Applications", pp. 208-227, AOCS Press, Champaign, IL, 1995]. While many grades and forms are available, deoiled lecithin produces the most consistent results. Typical commercially available examples are Ultralec P, Ultralec F and Ultralec G (Archer Daniels Midland, Decatur, IL) or Precept 8160, a powdered, enzyme-modified lecithin (Central Soya, Fort Wayne, IN).

A variety of sterols and their ester derivatives can be added to lecithin to enhance the aqueous dispersibility in the gut in the presence of bile salts and bile phospholipid. While cholesterol has frequently been used for this purpose, its absorption can lead to elevated LDL-cholesterol levels, making it a poor choice for the pharmaceutical applications contemplated here. Plant-derived sterols, especially those derived from soy and tall oil, are used in the present invention since they have been shown to lower LDL-cholesterol and they are considered to be safe [Jones, P.J.H., McDougall, D.E., Ntanios, F., & Vanstone, C.A. (1996) Dietary phytosterols as cholesterol-lowering agents in humans. Can. J. Physiol. Pharmacol. 75, 227]. Specifically, this invention contemplates the use of mixtures including, but not limited to sitosterol, campesterol, stigmasterol and brassicasterol and their corresponding fatty acid esters prepared as described elsewhere (Wester I., et al., "Stanol Composition and the use thereof", WO 98/06405). The reduced forms of the above-mentioned sterols and their corresponding esters are the most preferred, since they also lower human LDL-cholesterol and their absorption is from five- to ten-fold less than that of their non-reduced counterparts [Ostlund, R.E., et al., (2002), Am. J. of Physiol., 282, E 911; Spilburg et al., 4th International Symposium on the Role of Soy in Preventing and Treating Chronic Disease, November 4-7, 2002, San Diego, CA Abstract D4].

Hydrophobic drugs or potential drugs may be selected from any therapeutic class including but not limited to anesthetics, anti-asthma agents, antibiotics, antidepressants, anti-diabetics, anti-epileptics, antifungals, anti-gout, anti-neoplastics, anti-obesity agents, anti-protozoals, anti-phyretics, anti-virals, antipsychotics, calcium regulating agents, cardiovascular agents corticosteroids, diuretics, dopaminergic agents, gastrointestinal agents, hormones (peptide and non-peptide), immunosuppressants, lipid regulating agents, phytoestrogens, prostaglandins, relaxants and stimulants, vitamins/nutritionals and xanthines. A number of criteria can be used to determine appropriate candidates for this formulation system, including but not limited to the following: drugs or organic compounds that are known to be poorly dispersible in water, leading to long dissolution times; drugs or organic compounds that are known to produce a variable biological response from dose to dose or; drugs or organic compounds that have been shown to be preferentially soluble in hydrophobic solvents as evidenced by their partition coefficient in the octanol water system.

In addition to these components, other ingredients may be added that provide beneficial properties to the final product, such as vitamin E to maintain stability of the active species.

All the components are dissolved in a suitable non-polar organic solvent, such as chloroform, dichloromethane, ethyl acetate, pentane, hexane and heptane. The choice of solvent is dictated by the solubility of the components and the stability of the drug at the boiling point of the solvent. The preferred solvents are non-chlorinated and for heat stable compounds, heptane is the most preferred solvent because of this high boiling point, which increases the overall solubility of all the components.

The weight ratio of the components in the final mixture depends on the nature of the hydrophobic compound. The weight ratio of lecithin to the stanol/drug combination can vary form 0.45 to 10.0.

After all the components are dissolved at the desired ratio in the appropriate solvent, the liquid is removed at elevated temperature to maintain the solubility of all the components. Residual solvent can be removed by pumping under vacuum. Alternatively, the solvent can be removed by atomization as described in U.S. Patents 4,508,703 and 4,621,023. The solid is then added to water at a temperature that is less than the decomposition temperature of one of the components or the boiling point of water, whichever is lower. The mixture is vigorously mixed in a suitable mixer to form a milky solution, which is then homogenized, preferably with a sonicator, Gaulin dairy homogenizer or a microfluidizer. The water is then removed by spray drying, lyophilization or some other suitable drying method. Before drying, it is helpful, but not necessary, to add maltrin, starch, silicon dioxide or calcium silicate to produce a flowable powder that has more desirable properties for filling capsules or compression into tablets.

There are other known methods that can be used to prepare tablets. After the components have been mixed at the appropriate ratio in organic solvent, the solvent can be removed as described above. The solid material so prepared can then be compressed at elevated pressure and extruded into a rope. The rope can be cut in segments to form tablets. This method is similar to that described in U.S. Patent 6,312,703, but the inventor did not recognize the importance of pre-mixing the components in organic solvent. While this previous method produces a tablet, the components may not be as freely dispersible in bile salt and phospholipid when they are not pre-mixed in organic solvent. Alternatively, the solid material that results from homogenization and spray drying can be compressed at high pressure and extruded to form a rope that can be cut into tablets.

The precise details of tableting technique are not a part of this invention, and since they are well-known they need not be described herein in detail. Generally, pharmaceutical carriers which are liquid or solid may be used. The preferred liquid carrier is water. Flavoring materials may be included in the solutions as desired.

Solid pharmaceutical carriers such as starch, sugar, talc, mannitol and the like may be used to form powders. Mannitol is the preferred solid carrier. The powders may be used as such for direct administration to a patient, or instead, the powders may be added to suitable foods and liquids, including water, to facilitate administration.

The powders also may be used to make tablets, or to fill gelatin capsules. Suitable lubricants like magnesium stearate, binders such as gelatin, and disintegrating agents like sodium carbonate in combination with citric acid may be used to form the tablets.

While not precisely knowing why, and not wishing to be bound by any theory of operability, the fact is that for difficultly soluble drugs this composition and combination of steps achieved higher absorption rates, and at the same time has a beneficial effect on lowering cholesterol for those in need of it.

### EXAMPLE

*Preparation of Formulated Cyclosporin.* Cyclosporin A (0.50 gm) Ultralec (1.00 gm) and soy stanols (0.50) were mixed in a 30 mL Corex glass tube. Ethyl acetate (5.0 mL) was added to the tube and the mixture was warmed on a water bath of 60°C until all the solids dissolved. The clear solution was mixed thoroughly with a vortexer and the solvent was removed under a stream of nitrogen, with occasional warming to 60°C to enhance the removal of ethyl acetate solvent. Residual solvent was removed from the solid under vacuum. After the sample was thoroughly dried, water (10 mL) was added and the mixture was sonicated for four minutes to produce a creamy solution. Maltrin (500 mg) was dissolved in 3 mL of water and added to the creamy solution with mixing. After removing an aliquot for particle size analysis, the remaining solution was frozen in a dry ice acetone bath and lyophilized. An aliquot of the lyophilized material was redissolved in water and the particle size distribution of this re-hydrated material was determined and compared to that of the sonicated mixture from which it was derived. As shown in the Table below, the particle size distribution of the rehydrated sample indicates that drying and rehydration do not alter significantly the particle size distribution when compared to that of the starting material.

| Preparation | D[v,0.1]* | D[v,0.5]* | D[v,0.9]* |
|---|---|---|---|
| Hydrated Formulated Cyclosporin | 4.13 | 14.20 | 45.04 |
| Emulsion Dried and Rehydrated | 4.05 | 9.90 | 26.58 |

| | | | |
|---|---|---|---|
| *10% of the particles have a particle size less than this value in µm. The other parameters refer to the particle size for 50% and 90% of the particles, respectively. | | | |

*Preparation of Capsules Containing Formulated Solid Cyclosporin.* Formulated Cyclosporin A (125 mg), starch (75 mg), CaCO₃ (50 mg) and SiO₂ (3 mg) were mixed together and packed into a #1 gelatin capsule. When the gelatin capsule was added with stirring to 37°C water, the powder dispersed within 10 minutes after the capsule dissolved.

*Assessment of Bioavailability in Dogs.* Two dogs were dosed with 25 mg of Neoral capsules (Sandimmune) and two dogs were given 25 mg of encapsulated formulated Cyclosporin A (1.25 mg/kg/day). At 0, 1, 2, 4, 8, 12 and 24 hours post administration, blood was drawn into tubes containing EDTA.
After a washout period of at least 72 hours, the animals were given the alternate dose and the blood draws were repeated at the same time intervals. When all the samples were collected, they were assayed for Cyclosporin, using the Cyclo-Trac SP assay (Diasorin, Stillwater, MN). When cyclosporin A was formulated in this way, the area under the blood concentration-time curve was about 67% of that found for Neoral administration. The peak concentration of the blood concentration-time curve occurred at 4 hours for the formulated cyclosporin versus 2 hours for Neoral, reflecting a longer dissolution time of the solid.

It should be understood that certain modifications should be and will be apparent to those of ordinary skill in the art of pharmacology, and that such modifications to the precise procedures and compositions set forth herein are intended to come within the scope of the invention, either literally or by the Doctrine of Equivalents. In this light, the following claims are made.

## Claims

1. A solid drug delivery composition for normally difficulty soluble hydrophobic drug actives, comprising:
lecithin or an active derivative thereof;
a plant derived sterol (stanol) or ester derived from the sterol (stanol); and
an affective amount of a hydrophobic drug active,
wherein the weight ratio of lecithin or active derivative of lecithin to the combination of the sterol (stanol) or ester derived from the sterol (stanol) and drug active is from 0.20:1.0 to 1:1, said composition being solid.

2. The composition of claim 1 wherein an active derivative of lecithin is used, and the derivative is lysolecithin.

3. The drug delivery composition of claim 1 wherein the lecithin or active derivative of lecithin is derived from food material selected from the group consisting of soy, egg, or other recognized food or pharmaceutical grade commercial sources of lecithin material.

4. The drug delivery composition of claim 1 wherein the plant derived sterol (stanol) ester, derived from a vegetable oil source.

5. The composition of claim 1 wherein the drug delivery composition includes as an additional hydrophobic compound, vitamin E.

6. A method of preparing a drug delivery system for normally difficulty soluble hydrophobic drug actives, comprising:
mixing i) an effective amount of a hydrophobic drug active, ii) a phospholipid material selected from the group consisting of lecithin and active derivates of lecithin, and iii) a plant derived sterol (stanol) or esters derived from plant sterol (stanol) in which the fatty acid ester moiety is derived from a vegetable oil, with iv) a non-polar organic solvent; wherein the weight ratio of lecithin or active derivative of lecithin to the combination of the sterol (stanol) or ester derived from the sterol (stanol) and the drug active is from 0.20:1.0 to 1:1;
removing the solvent to leave a solid residue of the mixed component's;
adding water to the solid residue of the mixed components at a temperature less than the decomposition temperature of any one of the mixed components;
homogenizing the aqueous mixture;
drying the homogenized mixture; and
providing the dried solid residue of the mixed components in a solid pharmaceutical carrier format.

7. The method of claim 6 wherein the phospholipid material is lecithin.

8. The method of claim 6 wherein the phospholipid material is lysolecithin.

9. The method of claim 6 wherein the non-polar organic solvent is selected from the group consisting of ethyl acetate and heptane.

10. The method of claim 6 wherein the non-polar organic solvent is at its boiling point.

11. The method of claim 6 wherein the non-polar organic solvent is removed by elevating the temperature above the solvent's boiling point.

12. The method of claim 6 wherein the dried solid residue of the mixed components is dispersed in water with vigorous stirring at a temperature less than the decomposition temperature of any of the mixed components.

13. The method of claim 6 wherein the non-polar organic solvent is selected from the group consisting of heptane, chloroform, dichloromethane, and isopropanol.

14. The method of claim 6 wherein the solvent removal continues until a solid residue that contains less than 0.5% solvent is provided.

15. The method of claim 6 wherein the solid formed after solvent removal is pulverized to produce a dispersible powder.

16. The method of claim 6 wherein the powder from claim 15 is added with vigorous stirring to water at a temperature that is less than the decomposition temperature of one of the components or the boiling point of water, whichever is lower.

17. The method of claim 6 wherein water is introduced directly to the un-pulverised dried solid residue.

18. The method of claim 17 wherein the water is at a temperature that is less than the decomposition temperature of any one of the mixed components or the boiling point of water, whichever is lower.

19. The method of claim 6 wherein the aqueous mixture is homogenized in a homogenizer selected from the group consisting of a Gaulin homogenizer, a French press, a sonicator, and a microfluidizer.

20. The method of claim 6 wherein the homogenized aqueous mixture is dried in a drier selected from the group consisting of spray driers and lyophilizers.

21. The method of claim 20 wherein a drying aid selected from the group consisting of maltrin, starch, silicon dioxide and calcium silicate is added.

22. The method of claim 21 wherein the solid is converted into a tablet or capsule.

23. A solid product that is formed from the method in claim 15 by subjecting the material to compression or extrusion for at least 15 seconds at a pressure of at least 689500 Pa (100 pig).

24. A solid product that is formed from the method in claim 20 by subjecting the material to compression or extrusion for at least 15 seconds at a pressure of at least 689500 Pa (100 psig).

## Patentansprüche

1. Eine Zufuhrzusammensetzung fester Arzneistoffe für normalerweise schwerlösliche, hydrophobe Arzneimittelwirkstoffe, die aus folgendem bestehen:
Lecithin oder einem wirksamen Derivat desselben;
einem pflanzlichen Sterol (Stanol) oder aus dem Sterol gewonnenen Ester (Stanol); und
einer wirksamen Menge eines hydrophoben Arzneimittelwirkstoffes,
in dem das Gewichts-verhältnis von Lecithin oder wirksamen aus Lecithin gewonnenen Derivat zur Zusammen-setzung des Sterols (Stanol) oder aus dem Sterol (Stanol) gewonnenen Ester und Arznei-mittelwirkstoff von 0,20:1,0 bis 1:1 beträgt, wobei besagte Zusammensetzung fest ist.

2. Die Zusammensetzung nach Anspruch 1, in der ein wirksames Lecithinderivat verwendet wird und das Derivat Lysolecithin ist.

3. Die Arzneimittelzufuhr-Zusammensetzung nach Anspruch 1, in dem das Lecithin oder das wirksame, aus Lecithin gewonnenen Derivat, aus Lebensmittelmaterial aus der Gruppe selektiert wird, die aus folgendem besteht: Soja, Eier oder Lecithinmaterial aus anderen anerkannten handelsüblichen Quellen für Lebensmittel- oder Pharmaqualität.

4. Die Arzneimittelzufuhr-Zusammensetzung nach Anspruch 1, in dem der pflanzliche Sterolester (Stanol) aus einer Quelle pflanzlichen Öls gewonnen wird.

5. Die Zusammensetzung nach Anspruch 1, in dem die Arzneizufuhr-Zusammensetzung Vitamin E als eine zusätzliche hydrophobe Verbindung aufweist.

6. Eine Methode zur Herstellung eines Arzneimittel-Zufuhrsystems für normalerweise schwerlösliche, hydrophobe Arzneimittelwirkstoffe, die aus folgendem bestehen:
i) Mischung einer wirksamen Menge eines hydrophoben Arzneimittelwirkstoffes,
ii) eines Phospholipidmaterials, das aus der Gruppe selektiert wird, die aus Lecithin und wirksamen Lecithinderivaten besteht und
iii) einem pflanzlichem Sterol (Stanol) oder Ester, die aus pflanzlichem dem Sterol, (Stanol) gewonnenen werden, in dem der Fettsäureester-Anteil aus einem pflanzli-chem Öl gewonnen wird, mit
iv) einem unpolaren organischen Lösemittel, in dem das Gewichtsverhältnis des Lecithins oder wirksamen Lecithinderivats zu der Verbindung des Sterols (Stanol) oder aus dem Sterol (Stanol) gewonnenen Ester und des Arzneimittelwirkstoffs von 0,20:1,0 bis 1:1 beträgt,
der Entfernung des Lösemittels, um einen festen Rückstand der gemischten Komponenten zu hinterlassen;
Wasser zum festen Rückstand der gemischten Komponenten bei einer Temperatur hinzufü-gen, die niedriger als die Zersetzungstemperatur einer dieser gemischten Komponenten ist;
die wässerige Mischung homogenisieren;
die homogenisierte Mischung trocknen und
den getrockneten, festen Rückstand der gemischten Komponenten in einer festen pharma-zeutischen Trägerform bereitzustellen.

7. Die Methode nach Anspruch 6, in dem das Phospholipidmaterial Lecithin ist.

8. Die Methode nach Anspruch 6, in dem das Phospholipidmaterial Lysolecithin ist.

9. Die Methode nach Anspruch 6, in dem das unpolare organische Lösemittel aus der Gruppe gewählt wird, die aus Ethylacetat und Heptan besteht.

10. Die Methode nach Anspruch 6, in dem das unpolare organische Lösemittel an seinem Siedepunkt ist.

11. Die Methode nach Anspruch 6, in dem das unpolare organische Lösemittel entfernt wird, indem die Temperatur über den Siedepunkt des Lösemittels erhöht wird.

12. Die Methode nach Anspruch 6, in dem der getrocknete feste Rückstand der gemischten Komponenten in Wasser durch kräftiges Rühren bei einer Temperatur, die niedriger als die Zersetzungstemperatur einer dieser gemischten Komponenten ist, dispergiert wird.

13. Die Methode nach Anspruch 6, in dem das unpolare organische Lösemittel aus der Gruppe gewählt wird, die aus Heptan, Chloroform, Dichlormethan und Isopropanol besteht.

14. Die Methode nach Anspruch 6, in dem die Entfernung des Lösemittels weiter geführt wird, bis ein fester Rückstand bereitgestellt wird, der weniger als 0,5% Lösemittel enthält.

15. Die Methode nach Anspruch 6, in dem der nach der Lösemittelentfernung gebildete Feststoff pulverisiert wird, um ein dispergierbares Pulver zu erzeugen.

16. Die Methode nach Anspruch 6, in dem das Pulver aus Anspruch 15 unter kräftigem Rühren zum Wasser bei einer Temperatur hinzu gegeben wird, die niedriger als die Zersetzungstemperatur einer der gemischten Komponenten oder des Siedepunkts des Wassers ist, je nachdem welche niedriger ist.

17. Die Methode nach Anspruch 6, in dem Wasser direkt zum unpulverisierten trockenen, festen Rückstand eingeführt wird.

18. Die Methode nach Anspruch 17, in dem das Wasser eine Temperatur hat, die niedriger als die Zersetzungstemperatur einer der gemischten Komponenten oder des Siedepunkts des Wassers ist, je nachdem welche niedriger ist.

19. Die Methode aus Anspruch 6, in dem die wässrige Mischung in einem Homogeni-sator homogenisiert wird, der aus der Gruppe gewählt wird, die aus einem Gaulin Homo-genisator, einer French Press, einem Sonicator und einem Microfluidizer besteht.

20. Die Methode nach Anspruch 6, in dem die homogenisierte, wässrige Mischung in einem Trockner getrocknet wird, der aus der Gruppe gewählt wird, die aus Sprühtrocknern und Gefriertrocknern besteht.

21. Die Methode nach Anspruch 20, in dem ein Trockenmittel aus der Gruppe gewählt wird, die aus Maltrin, Stärke, Siliciumdoxid besteht und Calciumsilicat hinzugefügt wird.

22. Die Methode nach Anspruch 21, in dem der Feststoff zu einer Tabelle oder Kapsel gepresst wird.

23. Ein festes Produkt, das entsprechend der Methode aus Anspruch 15 hergestellt wird, indem das Material einem Komprimier- oder Extrudiervorgang wenigstens 15 Sekunden bei einem Druck von wenigstens 689500 Pa (100 psig) unterzogen wird.

24. Ein festes Produkt, das entsprechend der Methode aus Anspruch 20 hergestellt wird, indem das Material einem Komprimier- oder Extrudiervorgang wenigstens 15 Sekunden bei einem Druck von wenigstens 689500 Pa (100 psig) unterzogen wird.

## Revendications

1. Composition de délivrance de médicaments solides pour des substances médicamenteuses actives hydrophobes, normalement difficilement solubles, comprenant :
lécithine ou un dérivé actif de cette molécule ;
stérol (stanol) d'origine végétale ou un ester dérivé du stérol (stanol) ; et
quantité efficace d'une substance médicamenteuse active,
dans laquelle le rapport en poids entre la lécithine ou un dérivé actif de la lécithine, et la combinaison du stérol (stanol) ou de l'ester dérivé du stérol (stanol) et de la substance médicamenteuse active est compris entre 0,20:1,0 et 1:1, ladite composition étant solide.

2. Composition selon la revendication 1, dans laquelle est utilisé un dérivé actif de la lécithine, et le dérivé est la lysolécithine.

3. Composition de délivrance de médicaments selon la revendication 1, dans laquelle la lécithine ou le dérivé actif de la lécithine est obtenu à partir de matières comestibles sélectionnées dans le groupe qui inclut : soja, oeufs ou autres sources commerciales reconnues de lécithine de qualité alimentaire ou pharmaceutique.

4. Composition de délivrance de médicaments selon la revendication 1, dans laquelle l'ester de stérol (stanol) est obtenu à partir d'une source d'huile végétale.

5. Composition selon la revendication 1, dans laquelle la composition de délivrance de médicaments comprend la vitamine E à titre de composé hydrophobe additionnel.

6. Procédé de préparation d'un système de délivrance de médicaments pour des substances médicamenteuses actives hydrophobes, normalement difficilement solubles, comprenant :
mélange i) d'une quantité efficace d'une substance médicamenteuse active hydrophobe, ii) d'un phospholipide sélectionné dans le groupe qui comprend la lécithine et des dérivés actifs de la lécithine et iii) d'un stérol (stanol) d'origine végétale ou d'esters dérivés du stérol (stanol) d'origine végétale, dans lequel le fragment moléculaire ester d'acide gras est dérivé d'une huile végétale, avec iv) un solvant organique non polaire ; procédé selon lequel le rapport en poids de la lécithine ou du dérivé actif de la lécithine d'une part, et de la combinaison de stérol (stanol) ou d'ester dérivé du stérol (stanol) et de la substance médicamenteuse active d'autre part, égale entre 0,20:1,0 et 1:1 ;
extraction du solvant pour laisser un résidu solide des composants mélangés ;
ajout d'eau au résidu solide des composants mélangés à une température inférieure à la température de décomposition de l'un quelconque des composants mélangés ;
homogénéisation du mélange aqueux ;
séchage du mélange homogénéisé ; et
préentation du résidu solide des composants mélangés sous une forme galénique à vecteur pharmaceutique solide.

7. Procédé selon la revendication 6, selon lequel le phospholipide est la lécithine.

8. Procédé selon la revendication 6, selon lequel le phospholipide est la lysolécithine.

9. Procédé selon la revendication 6, selon lequel le solvant organique non polaire est sélectionné dans le groupe qui comprend acétate d'éthyle et heptane.

10. Procédé selon la revendication 6, selon lequel le solvant organique non polaire est à son point d'ébullition.

11. Procédé selon la revendication 6, selon lequel le solvant organique non polaire est extrait par élévation de la température au-dessus du point d'ébullition du solvant.

12. Procédé selon la revendication 6, selon lequel le résidu solide séché des composants mélangés est dispersé dans l'eau avec agitation vigoureuse, à une température inférieure à la température de décomposition de l'un quelconque des composants mélangés.

13. Procédé selon la revendication 6, selon lequel le solvant organique non polaire est sélectionné dans le groupe qui comprend heptane, chloroforme, dichlorométhane et isopropanol.

14. Procédé selon la revendication 6, selon lequel l'extraction du solvant se poursuit jusqu'à l'obtention d'un résidu solide qui contient moins de 0,5% de solvant.

15. Procédé selon la revendication 6, selon lequel le solide formé après l'extraction du solvant est pulvérisé pour produire une poudre dispersable.

16. Procédé selon la revendication 6, selon lequel la poudre selon la revendication 15 est ajoutée à l'eau, avec agitation vigoureuse, à une température qui est inférieure à la température de décomposition de l'un des composants, ou au point d'ébullition de l'eau, selon celui qui est le plus bas.

17. Procédé selon la revendication 6, selon lequel l'eau est introduite directement dans le résidu solide séché non pulvérisé.

18. Procédé selon la revendication 17, selon lequel l'eau est à une température qui est inférieure à la température de décomposition de l'un quelconque des composants mélangés ou au point d'ébullition, selon celui qui est le plus bas.

19. Procédé selon la revendication 6, selon lequel le mélange aqueux est homogénéisé dans un homogéniseur sélectionné dans le groupe qui comprend un homogéniseur Gaulin, une presse de laboratoire à piston, un sonicateur et un microfluidiseur.

20. Procédé selon la revendication 6, selon lequel le mélange aqueux homogénéisé est séché dans un sécheur sélectionné dans le groupe qui comprend les sécheurs par pulvérisation et les lyophiliseurs.

21. Procédé selon la revendication 20, selon lequel est ajoutée une aide au séchage sélectionnée dans le groupe qui comprend maltrine, amidon, dioxyde de silicium (silice cristalline) et silicate de calcium.

22. Procédé selon la revendication 21, selon lequel le solide est formé en un comprimé ou une gélule.

23. Produit solide qui est formé selon le procédé de la revendication 15 en soumettant la matière, pendant au moins 15 secondes, à une compression ou à une extrusion sous une pression minimum de 689500 Pa (100 psig).

24. Produit solide qui est formé selon le procédé de la revendication 20 en soumettant la matière, pendant au moins 15 secondes, à une compression ou à une extrusion sous une pression minimum de 689500 Pa (100 psig).
